# EUROPEAN PATENT SPECIFICATION

(11) **EP 3 538 043 B1**
(45) Date of publication and mention of the grant of the patent: **04.05.2022**
(21) Application number: 17801811.5
(22) Date of filing: 07.11.2017
(51) Int. Cl.: A61F 9/06

(54) **FLAT AUTOMATIC DARKENING FILTER AND WELDING PROTECTOR**
FLACHER AUTOMATISCHER VERDUNKELUNGSFILTER UND SCHWEISSSCHUTZ
FILTRE PLAT AUTOMATIQUE D'ASSOMBRISSEMENT ET DE PROTECTION DE SOUDAGE

(30) Priority: 08.11.2016 EP 16197666
(43) Date of publication of application: 18.09.2019
(73) Proprietor: 3M Innovative Properties Company, Saint Paul, Minnesota 55133-3427 (US)
(72) Inventor: MAGNUSSON, Kristina M., S-191 89 Sollentuna (SE); JAREFORS, Kenneth O.R., S-191 89 Sollentuna (SE); ZURAVSKAJA, Larissa, S-191 89 Sollentuna (SE); JACQUIER, Sébastien L.M., S-191 89 Sollentuna (SE)
(74) Representative: Vollmers, Hans-Gerd
(86) International application number: PCT/US2017/060293
(87) International publication number: WO 2018/089327

(56) References cited:
- WO-A1-95/29428
- WO-A1-2004/053586
- US-A1- 2014 168 546
- US-A1- 2014 168 546

## Description

### Field of the Invention

The invention relates to a flat automatic darkening filter for welding protection having a liquid crystal cell made of ultrathin glass substrates. The invention relates further to a welding protector having a flat automatic darkening filter.

### Background Art

Automatic darkening filters commonly have a switchable filter that automatically changes from a light-transmission-state to a dark-transmission-state in response to incident light. The switching is generally achieved through use of a photodetector that is located on, or as part of, personal protective equipment. The photodetector recognizes the presence of the incident light-to-be-filtered, and an electronic module generates a control voltage that, when applied to the switchable filter, causes the filter to change from the light-transmission-state to the dark-state.

Automatic light filters have been designed which contain liquid-crystal cells located between polarizing films. U.S. Patent No. 4,240,709 to Homell describes a switchable filter that has a single-twisted, nematic, liquid-crystal cell sandwiched between a pair of mutually crossed polarizers. The liquid-crystal cells are optically-transparent glass substrates that include transparent electrode and alignment layers. The liquid-crystal molecules orientate themselves in a particular direction when a voltage is applied across the liquid-crystal cell under the control of an electronic module. Many commercially available products use this kind of switchable filter.

US 2014/0168546 A1 discloses an automatic-darkening filter that comprises a first polarizer a second polarizer, a first liquid-crystal cell, and a sensor. The first polarizer has a first polarization direction, and the second polarizer has a second polarization direction. The liquid crystal cell is disposed between the first and second polarizers, and contains first and second optically-transparent, flexible, glass layers and with the liquid crystal layer being located between these layers. The sensor detects incident light and causes a signal to be sent, which causes molecular rotation within the liquid crystal layer.

WO 2004/053586 A1 discloses a liquid crystal shutter comprising at least two liquid crystal cells and at least three polarizers. Each of the liquid crystal cells contains a plurality of layers between two substrates and is disposed between two adjacent polarizers. The adjacent two liquid crystal cells have substantially equal absolute values and opposite directions of twist angles. The liquid crystal shutter of the invention substantially increases the viewing angle and the contrast ratio at large viewing angles, provides a symmetrical dependence of the contrast ratio on the viewing angle, and decreases the switching time as well as the driving voltages. The shutter provides substantially improved angular characteristics, high contrast ratio, good transparency in the light state, and a fast switching time.

The use of an automatic-darkening filter in a protective shield gives significant ergonomic benefits. Previously welders, for example, had to "nod" their welding shield down when they struck the welding arc to ensure that their eyes were protected from the torch light. Automatic welding filters eliminate this action since the welding shield can be left in position continuously.

### Summary of the Invention

The invention relates to an automatic darkening filter for welding protection, in particular to a flat, or generally planar, automatic darkening filter. The automatic darkening filter comprises a first liquid crystal cell having two flat, or generally planar, ultrathin glass substrates.

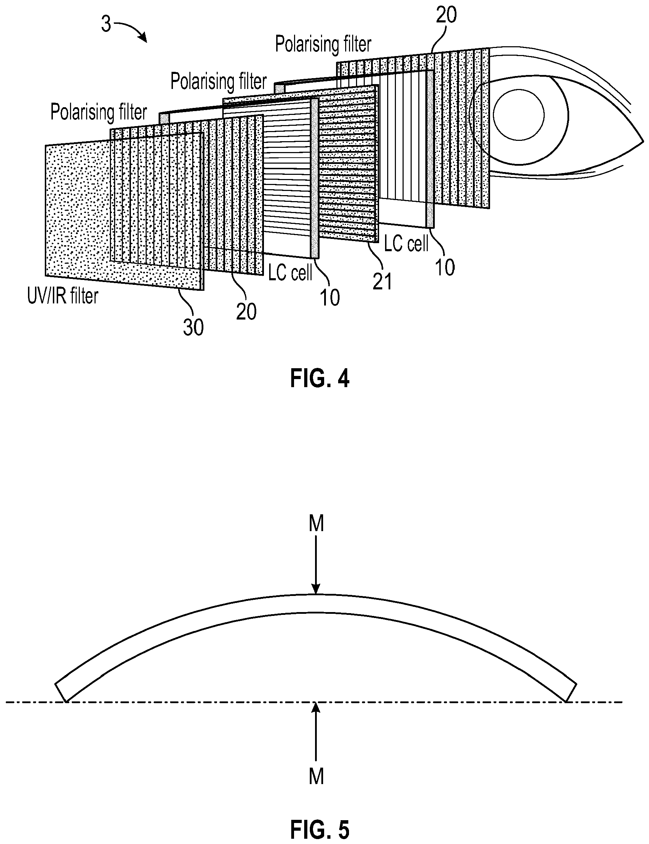

For the purpose of the present invention the term "ultrathin glass substrates" shall mean glass that has a thickness of between 30 µm and 200 µm, more preferably of between 50 µm and 150 µm and preferably about 100 µm. A glass substrate of the specified thickness typically provides for flexibility (in particular bendability) of the substrate. The flexibility provided of a glass substrate of a format of 120 x 80 mm at a thickness of 100 µm preferably enables a deflection of the longer side of the glass substrate by at least 30 mm. The deflection is measured in in the middle between the short sides of the glass substrate in a dimension of the thickness and between extreme points of the glass substrates in that dimension (see measuring point M in Fig. 5).

The invention is advantageous in that it provides a relatively inexpensive flat automatic darkening filter which is relatively lightweight but nevertheless mechanically stable and relatively aging resistant. It has been found that ultrathin glass unexpectedly is more resistant to mechanical impacts or shocks (and therefore exhibits a lower tendency to break from being exposed to mechanical impacts) than standard glass as used in prior art darkening filters. Although ultrathin glass has been used for curved darkening filters before it has been found advantageous for the mechanical resistance if the glass is used in a flat configuration. This may be based on the circumstance that curved filters are typically made by bending flat glass so that the curved glass is under a certain pretension. The invention further enables the making of a relatively lightweight welding protector. This helps maximizing the wearing comfort, for example.

In an embodiment the automatic darkening filter further comprises a first flat polarizer and a second flat polarizer. Each of the first and second polarizer have a polarization direction. The first and second polarizer being arranged in an overlapping manner on opposite sides of the first liquid crystal cell. Further, the first and second polarizer are arranged with their polarization directions oriented angularly offset to each other. Preferably the angular offset at which the first and second polarizer are arranged relative to each other is roughly 90 degrees. The angular offset at which the first and second polarizer are arranged relative to each other may particularly be within a range of 84 to 87 degrees.

In an embodiment the automatic darkening filter further comprises a second liquid crystal cell. The second liquid crystal cell may be identical to the first liquid crystal cell. In particular, the second liquid crystal cell may have two flat, or generally planar, ultrathin glass substrates. The automatic darkening filter may further comprise a third flat polarizer having a polarization direction. The second and the third polarizer are preferably arranged in an overlapping manner on opposite sides of the second liquid crystal cell. Further the first and the third polarizer are preferably arranged with their polarization directions oriented roughly parallel or parallel to each other. This means that preferably the third and second polarizer are arranged with their polarization directions oriented angularly offset to each other. Preferably the angular offset at which the third and second polarizer are arranged relative to each other is roughly 90 degrees or 90 degrees. The angular offset at which the second and the third polarizer are arranged relative to each other may for example be within a range of 84 to 87 degrees. Accordingly, the first and the third polarizer may be arranged with their polarization directions oriented within a range of 6 to 12 degrees relative to each other.

The first, second and third polarizer preferably each have two opposite major sides and a thickness defined between the major sides. Further, each of the glass substrates of the first and/or the second liquid crystal cell have two opposite major sides and a thickness defined between the major sides. The major sides of the glass substrates may fully or partially overlap the first, second and third polarizer. This means that the glass substrates may have a format which has two dimensions that are equal or larger than a corresponding dimension of a format of the polarizers. In this regard the term "format" refers to the two dimensions of the major sides. The glass substrates may further have a format which has two dimensions that are smaller than a corresponding dimension of a format of the polarizers. In the latter case the polarizers may serve as an edge protection for the glass substrates. It is noted that one of the two dimensions of the glass substrate be may generally equal to a corresponding dimension of the format of any or all of the polarizers and the other dimension of the format of the glass substrate may be greater or smaller than the corresponding dimension of the format of any or all of the polarizers.

In an embodiment each of the first and second liquid crystal cell have an edge sealing which seals the liquid crystals within a space formed by the glass substrates and the edge sealing.

In an embodiment each glass substrate comprising a transparent electrode layer and an alignment layer for the liquid crystals.

In an embodiment the ultrathin glass substrates have a thickness of between 30 µm and 200 µm. The electrode layer may be made of indium tin oxide and may have a thickness of about 10 nm to 50 nm, preferably about 20 nm. The alignment layer may be made of polyimide and may have a thickness of about 20 nm to 200 nm. Preferably, the thickness of the glass substrates refers to the thickness of only the glass although the electrode layer and the alignment layer may slightly add some thickness on the glass substrate.

In a preferred embodiment the automatic darkening filter has at least a third crystal cell, or a third and a fourth liquid crystal cell, or a third, fourth and a fifth liquid crystal cell. The third, fourth and fifth liquid crystal cell correspond in configuration to the first and second liquid crystal cell as described herein. Further, the automatic darkening filter may have a fourth polarizer, a fourth and a fifth polarizer or a fourth, fifth and sixth polarizer. For example, the automatic darkening filter may have a first, second, and third liquid crystal cell arranged between the first and the fourth polarizer. The second polarizer may be arranged between the first and the second liquid crystal cell and the third polarizer may be arranged between the second and the third liquid crystal cell.

In the embodiment of the automatic darkening filter having a fourth liquid crystal cell the first, second, third and fourth liquid crystal cell are preferably arranged between the first and the fifth polarizer and the fourth polarizer may be arranged between the third and the fourth liquid crystal cell.

In the embodiment of the automatic darkening filter having a fifth liquid crystal cell the first, second, third, fourth and fifth liquid crystal cell are preferably arranged between the first and the sixth polarizer and the fifth polarizer may be arranged between the fourth and the fifth liquid crystal cell.

An automatic darkening filter having up to five liquid crystal cells provides for a maximized darkening effect in the dark-state. In addition, such a darkening provides a maximized darkening effect even in the light-state although it allows a user to sufficiently see through. Thus, the safety for a user can be maximized. These embodiments are enabled due to the use of ultrathin glass substrates which result in an automatic darkening filter having up to five liquid crystal cells but still acceptable dimensions and weight.

In a further aspect the invention relates to a welding protector. Such a welding protector may be a welding shield or a welding helmet, for example. The welding protector comprises the automatic darkening filter of the invention.

In one embodiment the welding protector further comprises electronic circuitry having a power source and a light sensor and being electrically connected to the automatic darkening filter. The electronic circuitry is preferably configured for causing the automatic darkening filter to switch dependent on light detected by the light sensor. In particular, the electronic circuitry may be configured for causing the automatic darkening filter to switch to the dark-state in case the light sensor detects light exceeding a predetermined threshold light intensity. Further, the electronic circuitry may be configured for causing the automatic darkening filter to switch to the light-state in other cases than the case that the light sensor detects light exceeding the predetermined threshold light intensity. Accordingly the light-state may be a default state to which the automatic darkening filter resets. It is noted that the electronic circuitry is typically additionally configured for distinguishing the presence of a welding arc from the presence of other light, for example sun light. For example the electronic circuitry may be configured for recognizing a frequency or pulsation in the detected light. Such a frequency or pulsation may be present in a welding arc but not in sunlight, for example, so that the welding arc can be differentiated from the sun light.

The welding protector may further comprise a control panel via which a user, for example the welder, can adjust the threshold light intensity and, optionally, via which the user can switch the automatic darkening filter on or off.

The welding protector may further comprise headband by which a user can retain the welding protector on the user's head.

### Brief Description of the Figures

Fig. 1 is a perspective view of a welding protector, in particular of a welding helmet, according to an embodiment of the invention;
Fig. 2 is a cross-sectional view of a liquid crystal cell according to an embodiment of the invention;
Fig. 3 is a perspective view of two glass substrates with an edge sealing according to an embodiment of the invention;
Fig. 4 is an exploded view of an automatic darkening filter according to an embodiment of the invention; and
Fig. 5 illustrates a measurement of a deflection of a glass substrate.

### Detailed Description of the Invention

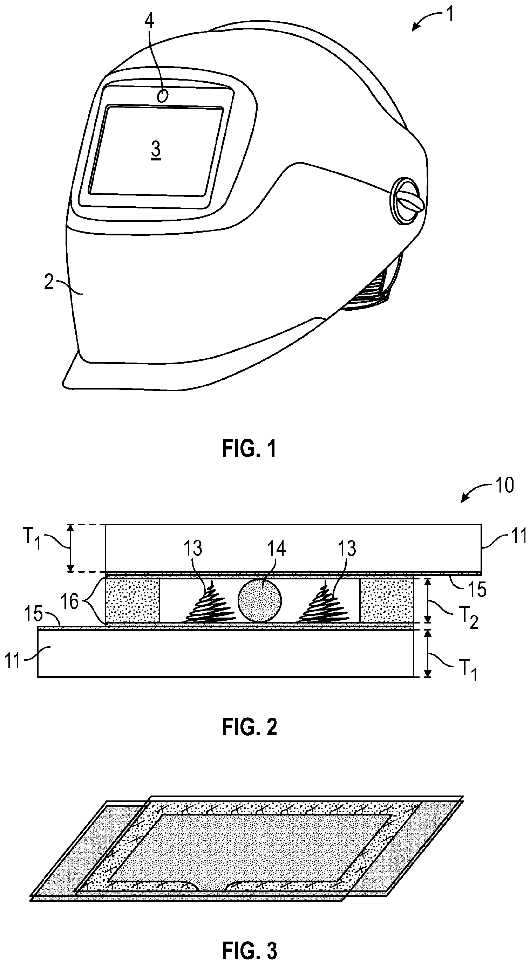

Fig. 1 shows a welding protector 1 which in the example is a welding helmet. The invention is however not limited to a use with a welding helmet, but may likewise be used with a welding shield or welding goggles in an appropriate configuration.

The welding protector 1 has a protective shield portion 2 for protecting a welder's face (and other head portions) from radiation, dust and splashes of hot materials as these may occur during welding. The welding protector 1 further has an automatic darkening filter 3 through which the welder can observe the welding arc during welding. In the example the automatic darkening is based on two liquid crystal cells by which the automatic darkening filter assembly 3 is electrically switchable between a light-state and a dark-state. When switched in the dark-state, the automatic darkening filter assembly 3 blocks a significant amount of light from being transmitted therethrough. This enables a user to observe a welding arc by seeing through the automatic darkening filter 3 without risking to be exposed to harmful light radiation from the welding arc. In the light-state the automatic darkening filter assembly 3 permits a significant amount of light to be transmitted therethrough. Thus, the automatic darkening filter assembly 3 in the light-state allows the user to see under ambient light conditions (in the absence of the welding arc).

The automatic darkening filter 3 comprises two (or more) liquid crystal cells that are arranged optically in sequence. This provides for multiplying the darkening effect (in particular in the dark-state) and thus a sufficient eye protection from light radiation.

Further, the welding protector 1 comprises at least one light sensor 4 and electronic circuitry (not illustrated) that causes the liquid crystal cells to switch dependent on light recognized by the light sensor(s). In particular, the light sensor 4 may provide a signal to the electronic circuitry depending on the light sensed by the light sensor 4. The signal provided by the light sensor 4 can typically be correlated to the intensity of light sensed by the light sensor 4. The electronic circuitry is set up to control the switching of the automatic darkening filter to the dark-state in case the light intensity (and optionally an additional frequency or pulsation) detected by the light sensor 4 exceeds a predetermined maximum value. Further, the electronic circuitry is set up to control the switching of the automatic darkening filter to the light-state in case the light intensity detected by the light sensor 4 drops below the predetermined maximum value.

Fig. 2 shows a liquid crystal cell 10 of the invention. The liquid crystal cell 10 has two glass substrates 11. Each glass substrate has opposite major sides and a thickness T1 defined between. In the example the glass substrates have a thickness of 100 µm. In addition, the liquid crystal cell 10 has a liquid crystal layer 12 which comprises liquid crystal molecules 13 and spacers 14. The liquid crystal layer 12 has a thickness T2 of 4 µm. The thickness T2 of the liquid crystal layer is determined by a gap between the major sides of the glass substrates and the size of the gap is provided by the spacers 14 arranged between the glass substrates. The spacers 14 are distributed across the major sides between the glass substrates. The amount of spacers may be between 30 and 200 spacers per square mm. Thus, the thickness of the liquid crystal layer 12 can be maintained relatively uniform across the liquid crystal cell 10. Accordingly, the darkening effect can be maintained relatively uniform across the liquid crystal cell 10 particularly in the dark-state. In the example the spacers are silica beads having a diameter of 4 µm.

Each glass substrate further has an electrode layer 15, which in the example is a transparent layer of indium tin oxide, as well as an alignment layer 16 for providing a default alignment of the liquid crystals.

Fig. 3 shows the two glass substrates of the liquid crystal cell shown in Fig. 2. An edge sealing 17 is provided in the margin of the glass substrate for sealing the gap between the glass substrates. The edge sealing hermetically seals the liquid crystals between the two glass substrates and provides mechanical stability (for example flexural resistance) for the liquid crystal cell.

Fig. 4 shows an exploded view of the automatic darkening filter 3. It is noted that the exploded view is a type of illustration only and that certain components that appear to be spaced from each other are normally mounted in contact to each other. The automatic darkening filter 3 comprises two liquid crystal cells 10 arranged optically in sequence with a (in the example horizontal) polarizer 21 arranged between. Further, the automatic darkening filter 3 comprises two (in the example vertical) polarizers 20 on the side of each liquid crystal cell 10 opposite of that side of the liquid crystal cell 1 0 on which the horizontal polarizer 21 is arranged. Thus, a sandwich arrangement is formed in which a vertical polarizer 20, a liquid crystal cell 10, a horizontal polarizer 21, a further liquid crystal cell and another vertical polarizer 20 are arranged in sequence. The skilled person will be aware that the vertical polarizers can be replaced by a horizontal polarizer and the horizontal polarizer can be replaced by a vertical polarizer as long as the polarizer in the middle is different from the outer polarizers Further, other orientations are possible as long as the polarizer in the middle provides for a polarization which orientation is inclined relative to the orientation of the polarization provided by the outer polarizers. Hence, in absence of any liquid crystals the combination of the three polarizers blocks light through the three polarizers to a significant level.

Further, the automatic darkening filter 3 has a UV (ultraviolet light) filter that typically also includes an IR (infrared light) filter. The UV light filter blocks at least a significant amount of ultraviolet light. The UV light filter is arranged on a side of the automatic darkening filter that faces away from a person's (for example a welder's) eye 100 who uses the automatic darkening filter 3. The UV filter is preferably fixedly laminated into the automatic darkening filter.

Furthermore, the automatic darkening filter 3 may comprise an exchangeable transparent protective layer on the eye facing side of the automatic darkening filter 3 and/or on the opposite side.

## Claims

1. An automatic darkening filter (3) for welding protection, comprising a first liquid crystal cell (10) having two flat ultrathin glass substrates (11), **characterized in that** the ultrathin glass substrates (11) have a thickness of between 30 µm and 200 µm.

2. The automatic darkening filter (3) of claim 1, further comprising a first and a second flat polarizer (20, 21) each having a polarization direction, the first and second polarizer (20, 21) being arranged in an overlapping manner on opposite sides of the first liquid crystal cell (10), with their polarization directions oriented angularly offset to each other.

3. The automatic darkening filter (3) of claim 2, further comprising a second liquid crystal cell having two flat ultrathin glass substrates.

4. The automatic darkening filter (3) of claim 3, further comprising a third flat polarizer having a polarization direction, the second and the third polarizer being arranged in an overlapping manner on opposite sides of the second liquid crystal cell, wherein the first and the third polarizer are arranged with their polarization directions oriented parallel or at an angle within a range of 6 to 12 degrees relative to each other.

5. The automatic darkening filter (3) of claim 3 or 4, wherein each of the first and second liquid crystal cell have an edge sealing which seals the liquid crystals within a space formed by the glass substrates and the edge sealing.

6. The automatic darkening filter (3) of claim 5, wherein each of the glass substrates (11) comprising a transparent electrode layer (15) and an alignment layer (16) for the liquid crystals.

7. The automatic darkening filter (3) of any of the preceding claims, wherein the ultrathin glass substrates (11) have a thickness of between 50 µm and 150 µm.

8. The automatic darkening filter (3) of claim 4, having a third liquid crystal cell and a fourth polarizer, wherein the first, second, and third liquid crystal cell are arranged between the first and the fourth polarizer, and wherein the second polarizer is arranged between the first and the second liquid crystal cell and the third polarizer is arranged between the second and the third liquid crystal cell.

9. The automatic darkening filter (3) of claim 8, having a fourth liquid crystal cell and a fifth polarizer, wherein the first, second, third and fourth liquid crystal cell are arranged between the first and the fifth polarizer and the fourth polarizer being arranged between the third and the fourth liquid crystal cell.

10. The automatic darkening filter (3) of claim 9, further having a fifth liquid crystal cell and a sixth polarizer, wherein the first, second, third, fourth and fifth liquid crystal cell are arranged between the first and the sixth polarizer and the fifth polarizer being arranged between the fourth and the sixth liquid crystal cell.

11. A welding protector (1), such as a welding shield or welding helmet, comprising the automatic darkening filter (3) of any of the preceding claims.

12. The welding protector (1) of claim 11, further comprising electronic circuitry having a power source and a light sensor and being electrically connected to the automatic darkening filter (3), wherein the electronic circuitry is configured for causing the automatic darkening filter (3) to switch dependent on light detected by the light sensor (4).

## Patentansprüche

1. Ein automatischer Verdunkelungsfilter (3) als Schweißschutz, umfassend eine erste Flüssigkristallzelle (10) mit zwei flachen ultradünnen Glassubstraten (11), **dadurch gekennzeichnet, dass** die ultradünnen Glassubstrate (11) eine Dicke zwischen 30 µm und 200 µm aufweisen.

2. Der automatische Verdunkelungsfilter (3) nach Anspruch 1, ferner umfassend einen ersten und einen zweiten flachen Polarisator (20, 21), die jeweils eine Polarisationsrichtung aufweisen, wobei der erste und der zweite Polarisator (20, 21) überlappend auf gegenüberliegenden Seiten der ersten Flüssigkristallzelle (10) angeordnet sind und ihre Polarisationsrichtungen winkelversetzt zueinander ausgerichtet sind.

3. Der automatische Verdunkelungsfilter (3) nach Anspruch 2, ferner umfassend eine zweite Flüssigkristallzelle mit zwei flachen ultradünnen Glassubstraten.

4. Der automatische Verdunkelungsfilter (3) nach Anspruch 3, ferner umfassend einen dritten flachen Polarisator, der eine Polarisationsrichtung aufweist, wobei der zweite und der dritte Polarisator überlappend auf gegenüberliegenden Seiten der zweiten Flüssigkristallzelle angeordnet sind, wobei der erste und der dritte Polarisator so angeordnet sind, dass ihre Polarisationsrichtungen parallel oder in einem Winkel innerhalb eines Bereichs von 6 bis 12 Grad relativ zueinander ausgerichtet sind.

5. Der automatische Verdunkelungsfilter (3) nach Anspruch 3 oder 4, wobei jede Zelle der ersten und zweiten Flüssigkristallzelle eine Randdichtung aufweist, welche die Flüssigkristalle innerhalb eines von den Glassubstraten und der Randdichtung gebildeten Raums abdichtet.

6. Der automatische Verdunkelungsfilter (3) nach Anspruch 5, wobei jedes der Glassubstrate (11) eine transparente Elektrodenschicht (15) und eine Ausrichtungsschicht (16) für die Flüssigkristalle umfasst.

7. Der automatische Verdunkelungsfilter (3) nach einem der vorstehenden Ansprüche, wobei die ultradünnen Glassubstrate (11) eine Dicke zwischen 50 µm und 150 µm aufweisen.

8. Der automatische Verdunkelungsfilter (3) nach Anspruch 4, aufweisend eine dritte Flüssigkristallzelle und einen vierten Polarisator, wobei die erste, zweite und dritte Flüssigkristallzelle zwischen dem ersten und dem vierten Polarisator angeordnet sind, und wobei der zweite Polarisator zwischen der ersten und zweiten Flüssigkristallzelle angeordnet ist und der dritte Polarisator zwischen der zweiten und dritten Flüssigkristallzelle angeordnet ist.

9. Der automatische Verdunkelungsfilter (3) nach Anspruch 8, aufweisend eine vierte Flüssigkristallzelle und einen fünften Polarisator, wobei die erste, zweite, dritte und vierte Flüssigkristallzelle zwischen dem ersten und dem fünften Polarisator angeordnet sind und der vierte Polarisator zwischen der dritten und der vierten Flüssigkristallzelle angeordnet ist.

10. Der automatische Verdunkelungsfilter (3) nach Anspruch 9, ferner aufweisend eine fünfte Flüssigkristallzelle und einen sechsten Polarisator, wobei die erste, zweite, dritte, vierte und fünfte Flüssigkristallzelle zwischen dem ersten und dem sechsten Polarisator angeordnet sind und der fünfte Polarisator zwischen der vierten und der sechsten Flüssigkristallzelle angeordnet ist.

11. Ein Schweißschutz (1), wie ein Schweißschild oder ein Schweißhelm, umfassend den automatischen Verdunkelungsfilter (3) nach einem der vorstehenden Ansprüche.

12. Der Schweißschutz (1) nach Anspruch 11, ferner umfassend eine elektronische Schaltung, die eine Energiequelle und einen Lichtsensor aufweist und elektrisch mit dem automatischen Verdunkelungsfilter (3) verbunden ist, wobei die elektronische Schaltung konfiguriert ist, den automatischen Verdunkelungsfilter (3) zu veranlassen, in Abhängigkeit von dem von dem Lichtsensor (4) erfassten Licht umzuschalten.

## Revendications

1. Filtre d'assombrissement automatique (3) pour une protection de soudage, comprenant une première cellule à cristaux liquides (10) comportant deux substrats en verre ultraminces plats (11), **caractérisé en ce que** les substrats en verre ultraminces (11) ont une épaisseur entre 30 µm et 200 µm.

2. Filtre d'assombrissement automatique (3) selon la revendication 1, comprenant en outre un premier et un deuxième polariseur plat (20, 21) ayant chacun une direction de polarisation, les premier et deuxième polariseurs (20, 21) étant agencés de manière chevauchante sur des côtés opposés de la première cellule à cristaux liquides (10), avec leurs directions de polarisation orientées angulairement décalées l'une par rapport à l'autre.

3. Filtre d'assombrissement automatique (3) selon la revendication 2, comprenant en outre une deuxième cellule à cristaux liquides comportant deux substrats en verre ultraminces plats.

4. Filtre d'assombrissement automatique (3) selon la revendication 3, comprenant en outre un troisième polariseur plat ayant une direction de polarisation, le deuxième et le troisième polariseur étant agencés de manière chevauchante sur des côtés opposés de la deuxième cellule à cristaux liquides, dans lequel le premier et le troisième polariseur sont agencés avec leurs directions de polarisation orientées parallèlement ou selon un angle inscrit dans une plage de 6 à 12 degrés l'une par rapport à l'autre.

5. Filtre d'assombrissement automatique (3) selon la revendication 3 ou 4, dans lequel chacune des première et deuxième cellules à cristaux liquides présente un scellement de bord qui scelle les cristaux liquides dans un espace formé par les substrats en verre et le scellement de bord.

6. Filtre d'assombrissement automatique (3) selon la revendication 5, dans lequel chacun des substrats en verre (11) comprenant une couche d'électrode transparente (15) et une couche d'alignement (16) pour les cristaux liquides.

7. Filtre d'assombrissement automatique (3) selon l'une quelconque des revendications précédentes, dans lequel les substrats en verre ultraminces (11) ont une épaisseur entre 50 µm et 150 µm.

8. Filtre d'assombrissement automatique (3) selon la revendication 4, comportant une troisième cellule à cristaux liquides et un quatrième polariseur, dans lequel les première, deuxième et troisième cellules à cristaux liquides sont agencées entre le premier et le quatrième polariseur, et dans lequel le deuxième polariseur est agencé entre la première et la deuxième cellule à cristaux liquides et le troisième polariseur est agencé entre la deuxième et la troisième cellule à cristaux liquides.

9. Filtre d'assombrissement automatique (3) selon la revendication 8, comportant une quatrième cellule à cristaux liquides et un cinquième polariseur, dans lequel les première, deuxième, troisième et quatrième cellules à cristaux liquides sont agencées entre le premier et le cinquième polariseur et le quatrième polariseur étant agencé entre la troisième et la quatrième cellule à cristaux liquides.

10. Filtre d'assombrissement automatique (3) selon la revendication 9, comportant en outre une cinquième cellule à cristaux liquides et un sixième polariseur, dans lequel les première, deuxième, troisième, quatrième et cinquième cellules à cristaux liquides sont agencées entre le premier et le sixième polariseur et le cinquième polariseur étant agencé entre la quatrième et la sixième cellule à cristaux liquides.

11. Protection de soudage (1), telle qu'une visière de soudage ou un casque de soudage, comprenant le filtre d'assombrissement automatique (3) selon l'une quelconque des revendications précédentes.

12. Protection de soudage (1) selon la revendication 11, comprenant en outre un montage de circuits électroniques présentant une source d'alimentation et un capteur de lumière et étant connecté électriquement au filtre d'assombrissement automatique (3), dans lequel le montage de circuits électroniques est configuré pour amener le filtre d'assombrissement automatique (3) à commuter en fonction de la lumière détectée par le capteur de lumière (4).
